# EUROPEAN PATENT SPECIFICATION

(11) **EP 0 833 842 B1**
(45) Date of publication and mention of the grant of the patent: **29.09.1999**
(21) Application number: 96920822.2
(22) Date of filing: 17.06.1996
(51) Int. Cl.: C07K 14/47, A61K 38/10, A61K 38/16

(54) **NOVEL PEPTIDES FOR USE IN TREATMENT OF T-CELL MEDIATED CARTILAGE DESTRUCTION IN AUTOIMMUNE DISEASES**
NEUE PEPTIDE ZUR VERWENDUNG BEI BEHANDLUNG VON DURCH T-ZELLEN VERMITTELTER KNORPELZERSTÖRUNGIN AUTOIMMUNKRANKHEITEN
NOUVEAUX PEPTIDES UTILISES DANS LE TRAITEMENT DE LA DESTRUCTION DU CARTILAGE INDUITE PAR LES LYMPHOCYTES T DANS LES MALADIES AUTO-IMMUNITAIRES

(30) Priority: 19.06.1995 EP 95201656
(43) Date of publication of application: 08.04.1998
(73) Proprietor: Akzo Nobel N.V., 6824 BM Arnhem (NL)
(72) Inventor: VERHEIJDEN, Gijsbertus, Franciscus, Maria, NL-5345 XT Oss (NL); BOOTS, Anna, Maria, Helena, NL-5366 AV Megen (NL)
(74) Representative: Ogilvie-Emanuelson, Claudia Maria
(86) International application number: EP9602605
(87) International publication number: WO9700270

(56) References cited:
- IMMUNOLOGY, vol. 78, no. 4, April 1993, OXFORD, ENGLAND, pages 586-591, XP002013660 J A GOODACRE ET AL.: "Human cartilage aggrecan CS1 region contians cryptic T-cell recognition sites"
- CLIN. IMMUNOL. IMMUNOPATHOL., vol. 64, no. 2, August 1992, NEW YORK, USA, pages 121-128, XP002013661 M G DANIELI ET AL.: "Juvenile rheumatoid arthritis patients manifest immune reactivity to the mycobacterial 65-kDa heat shock protein, to its 180-188 peptide, and to a partilaay homologous peptide of the proteoglycan link protein"
- BIOCH. SOC. TRANS., vol. 18, no. 5, October 1990, LONDON, page 955 XP002013662 N J GOODSTONE ET AL.: "Responses of T-cells from patients with inflammatory arthritis to human cartilage antigens"
- J CELL BIOLOGY, vol. 105, no. 5, November 1987, NEW YORK, pages 2403-2408, XP002013663 P F GOETINK ET AL.: "The tandemly repeated sequences of cartilage link protein contain the sites for interaction with hyaluronic acid" cited in the application
- JOURNAL OF BIOLOGICAL CHEMISTRY (MICROFILMS), vol. 261, no. 8, 15 March 1986, MD US, pages 353519-3535, XP002013685 P J NEAME ET AL.: "The primary structure of link protein from rat chondrosarchoma proteoglycan aggregate" cited in the application
- JOURNAL OF BIOLOGICAL CHEMISTRY, vol. 264, no. 10, 5 April 1989, MD US, pages 5981-5987, XP002013686 G PERIDES ET AL.: "Isolation and partial characterization of a glial hyaluronate-binding protein" cited in the application
- CHEMICAL ABSTRACTS, vol. 105, no. 23, 8 December 1986 Columbus, Ohio, US; abstract no. 2050003n, J P PERIN ET AL.: "Structural relationship between link proteins and proteoglycan monomers" page 241; XP002013687 & FEBS LETTERS, vol. 206, no. 1, 1986, AMSTERDAM NL, pages 73-77, cited in the application

## Description

The invention relates to novel peptides and their use in treatment of T-cell mediated articular cartilage destruction in autoimmune diseases.

More specifically, the invention relates to the use of said novel peptides in a peptide-induced tolerance therapy for the induction of tolerance to autoaggressive T cells associated with T-cell mediated articular cartilage destruction in autoimmune diseases, more specifically arthritis and in particular reumatoid arthritis.

The invention furthermore embraces pharmaceutical compositions comprizing said peptides and a diagnostic method for the detection of autoreactive T cells in a test sample, said T cells being associated with T-cell mediated articular cartilage destruction in autoimmune diseases and test kits to be used in said method.

The immune system is based on the discrimination between foreign antigens (non-self antigens) and autoantigens (self-antigens, derived from the individuals own body) achieved by a build in tolerance against the autoantigens.

The immune system protects individuals against foreign antigens and responds to exposure to a foreign antigen by activating specific cells such as T- and B lymphocytes and by producing soluble factors like cytokines, antibodies and complement factors. The antigen to which the immune system responds is processed by the antigen presenting cells (APCs) and a fragment of the antigen is expressed on the cell surface associated with a major histocompatibility complex (MHC) class II glycoprotein. The MHC-glycoprotein-antigen-fragment complex is presented to a T cell which by virtue of its T cell receptor recognizes the antigen fragment conjointly with the MHC class II protein to which it is bound. The T cell becomes activated, i.e. proliferates and/or produces cytokines, resulting in the expansion of the activated T cells directed to the antigen under attack (Grey et al., Sci. Am., 261 :3 8-46, 1989).

Self antigens are also continuously, processed and presented as antigen fragments by the MHC glycoproteins to T cells (Jardetsky et al., Nature 353:326-329, 1991). Under normal circumstances the immune system is tolerant to self antigens and activation of the immune response by these self antigens is avoided. Thus, self recognition is intrinsic to the immune system.

When tolerance to self antigens is lost, the immune system becomes activated against one or more self antigens, resulting in the activation of autoreactive T cells and the production of autoantibodies. This phenomenon is referred to as autoimmunity. As the immune response in general is destructive, i.e. meant to destroy the invasive foreign antigen, autoimmune responses can cause destruction of the body's own tissue.

The contribution of T cells to autoimmune diseases has been established by several studies. In mice, experimental autoimmune encephalomyelitis (EAE) is mediated by a highly restricted group of T cells, linked by their specificity for a single epitope of myelin basic protein (MBP) complexed to an MHC class II molecule. In the Lewis rat, a species with high susceptibility to various autoimmune diseases, disease has been shown to be mediated by T cells. In humans autoimmune diseases are also thought to be associated with the development of auto-aggressive T cells.

A destructive autoimmune response has been implicated in various diseases such as rheumatoid arthritis (RA), in which the integrity of articular cartilage is destroyed by a chronic inflammatory process resulting from the presence of large numbers of activated lymphocytes and MHC class II expressing cells. The mere presence of cartilage appears to be sufficient for sustaining the local inflammatory response: it has been shown that cartilage degradation is associated with the activity of cartilage-responsive autoreactive T cells in RA (Sigall et al., Clin. Exp. Rheumat. 6:59, 1988; Glant et al., Biochem. Soc. Trans. 18:796, 1990; Burmester et al., Rheumatoid arthritis Smolen, Kalden, Maini (Eds) Springer-Verlag Berlin Heidelberg, 1992). Furthermore, removal of cartilage from RA patients by surgery was shown to reduce the inflammatory process. The cartilage proteins are therefore considered to be target autoantigens which are competent of stimulating T cells. Activation of these autoreactive T cells leads to development of autoimmune disease.

The inflammatory response resulting in the destruction of the cartilage can be treated by steroid drugs. However, these drugs are immunosuppressive drugs that are nonspecific and have toxic side effects. The disadvantages of nonspecific immunosuppression makes this a highly unfavourable therapy.

An antigen-specific, nontoxic immunosuppression therapy would provide a very attractive alternative for the nonspecific immunosuppression. This antigen-specific therapy involves the treatment of patients with MHC Class II binding T-cell reactive peptides derived from the autoantigen. These MHC Class II binding T-cell reactive peptides correspond to T cell epitopes of the target autoantigen and can be used to induce specific T cell tolerance both to the administered peptides and the autoantigen. To effectively use the peptide-induced tolerance therapy to treat the T cell mediated articular cartilage destruction, there is a great need for MHC Class II binding T cell-reactive peptides which can desensitize patients against the self antigen that is activating the T cells responsible for the inflammatory process.

The present invention provides for such MHC Class II binding T-cell reactive peptides, that are suitable for use in a peptide-induced tolerance therapy for the induction of tolerance to autoaggressive T cells associated with T-cell mediated cartilage destruction in autoimmune diseases. More specifically, the present invention provides MHC Class II binding T-cell reactive peptides which are very suitable for use in a peptide-induced tolerance therapy for the induction of tolerance to autoaggressive T cells associated with T-cell mediated cartilage destruction in arthritis, in particular reumatoid arthritis.

The peptides according to the invention have an amino acid sequence of 13 to 55 amino acid residues, characterized in that said peptides compnse at least the amino acid sequence (SEQ ID No:1)

AGWLR₁DR₂R₃R₄R₅YPI

in which R₁ is A or S; R₂ is Q, R or G; R₃ is T of S; R₄ is V or L; and R₅ is R or Q.

More specifically, the peptides according to the invention compnse the amino acid sequence (SEQ ID No:2)

AGWLR₁DR₂R₃LR₅YPI

in which R₁ is A or S; R₂ is Q, R or G; R₃ is T or S; and R₅ is R or Q.

In particular, the peptides according to the invention compnse at least one of the amino acid sequences AGWLADQTVRYPI (SEQ ID No:3), AGWLADRSVRYPI (SEQ ID No:4), AGWLADGSLRYPI (SEQ ID No:5) or AGWLSDGSVQYPI (SEQ ID No:6) or combinations thereof.

Preferably the peptides according to the invention have an amino acid sequence of 13-35 amino acid residues, more preferably 13-25 amino acid residues. Highly preferred are peptides having an amino acid sequence of 13-19 residues. Particularly preferred are the peptides consisting of amino acid sequence AGWLADQTVRYPI (SEQ ID No:3), AGWLADRSVRYPI (SEQ ID No:4), AGWLADGSLRYPI (SEQ ID No:5) and AGWLSDGSVQYPI (SEQ ID No:6).

The peptides according to the invention are also understood to comprise multimers, such as for example a dimer or a trimer, which are build up from monomeric building blocks formed by the peptides according to the invention. These monomeric building blocks optionally can be separated by spacer residues. The multimers according to the invention have the advantage that they provide a multitude of T-cell reactive peptides.

The present invention resides in the fact that the peptides according to the invention resemble MHC Class II binding T-cell epitopes present on autoantigenic proteins which are constituents of the human articular cartilage. More specifically, the peptides according to the invention resemble MHC Class II binding T-cell epitopes present on the large aggregating proteoglycan of human articular cartilage, human aggrecan (HAG) and human cartilage link protein (HCLP).

It was surprisingly found that the amino acid residues 201-213, 299-311 and 623-635 of the primary structure of HAG (starting from the methionine in the signal sequence) as well as the amino acid residues 207-219 of the primary structure of HCLP (starting from the methionine in the signal sequence) display MHC Class II binding T-cell epitopes which are recognized by cartilage-responsive autoaggressive T cells associated with the destruction of articular cartilage in autoimmune diseases, more specific arthritis, in particular reumatoid arthntis. The primary structures of HAG and HCLP have been descnbed in Doege et al., J. Biol. Chem. Vol. 266, no. 2: 894-902 (1991) and Dudhia et al., Nucl. Acid Res., Vol. 18, no. 5: 1292 (1990) respectively.

Although articular cartilage proteins are considered to be the target autoantigens competent of stimulating autoaggressive T cells involved in the destruction of articular cartilage in autoimmune diseases, it was not untill the present invention that these MHC Class II binding T-cell epitopes associated with cartilage-responsive autoreactive T cells have been identified on the cartilage proteins, in particular on HAG and HCLP. The peptides according to the invention resemble these MHC Class II binding T-cell epitopes, thus providing T-cell reactive peptides which can be used in the peptide-induced T-cell tolerance therapy. Accordingly, patients can be treated with the peptides according to the invention to induce specific T-cell tolerance not only to the administered peptides but to the target autoantigens HAG and HCLP as wel. As other components of the immune system are not affected by the peptides according to the invention, the immune system of the patient will remain intact and will be able to protect the patient against other infections.

Thus, the peptides according to the invention provide a very attractive alternative for the classic steroid drugs in the treatment of T-cell mediated destruction of articular cartilage in autoimmune diseases, more specific arthritis, in particular reumatoid arhritis.

Peptides according to the invention have been descnbed. Perin et al, FEBS Letters 206:73 (1986) describes the structural relationship between link proteins and proteoglycan monomers and discloses a peptide fragment obtained after tryptic digestion of the link protein. The peptide fragment has the amino acid sequence SSAGWLADRSVRYPISKARPNXGG. Goetinck et al, J. Cell Biol. 105:2403-2408 (1987) discloses the peptides NAGWLSDGSVQYPITKPREP and DAGWLADGSVRYPISRPRKR which correspond to the amino acid residues Asn²⁰⁷- Pro²²⁶ and Asp³⁰⁶-Arg³²⁵ respectively of the primary structure of link protein. Said peptides were synthesized to study the interactions between link protein and hyaluronic acid and said amino acid residues were found to be involved in the binding of link protein to hyaluronic acid. Neame et al, J. Biol. Chem. 261 (8) :3519-3535, (1986) descnbes the elucidation of the primary structure of link protein from rat chondrosarcoma proteglycan aggragate. Analysis of a triptic digest of the link protein revealed a fragment having the amino acid sequence GGLDWCNAGWLSDGSVQYPITKPBR. Perides et al, J. Biol. Chem., Vol. 264, no. 10: 5981-5987 (1989) descnbes the isolation and partial characterization of a glial hyaluronate-binding protein (GHBP). Tryptic digestion of GHBP results in several peptide fragments, one of which having the amino acid sequence EQLFAAYEDGFEQCDAGWLADQTVRYPIRAPRVGCY.

None of these publications however disclose that said peptides display MHC Class II binding T-cell epitopes which are recognized by cartilage-responsive autoaggressive T cells involved in the destruction of articular cartilage in immune diseases. Nor do these publications suggest or hint towards the use of said peptides in a peptide-induced T-cell tolerance therapy for the induction of tolerance to autoaggressive T cells associated with T-cell mediated destruction of the articular cartilage in autoimmune diseases.

The peptides according to the invention can be prepared by well known organic chemical methods for peptide synthesis such as, for example, solid-phase peptide synthesis described for instance in J. Amer. Chem. Soc. 85:2149 (1963) and Int. J. Peptide Protein Res. 35:161-214 (1990).

The peptides according to the invention can also be prepared by recombinant DNA techniques. A nucleic acid sequence coding for a peptide according to the invention or a multimer of said peptides is inserted into an expression vector. Suitable expression vectors are, amongst others, plasmids, cosmids, virusses and YAC's (Yeast Artificial Chromosomes) which comprise the necessary control regions for replication and expression. The expression vector can be brought to expression in a host cell. Suitable host cells are, for instance, bacteria, yeast cells and mammalian cells. Such techniques are well known in the art, see for instance Sambrooke et al, Molecular Cloning:a Laboratory Manual, Cold Spring Harbor laboratory Press, Cold Spring Harbor, 1989.

According to the invention, patients suffering from T-cell mediated destruction of the articular cartilage can be treated with a therapeutical composition comprizing one or more peptides according to the invention and a pharmaceutical acceptable carrier. Administration of the pharmaceutical composition according to the invention will induce tolerance of the specific autoreactive T cells of these patients to the autoantigenic proteins in the articular cartilage under attack and other self antigens which display the identified MHC Class II binding T cell epitopes characterized by one of the amino acid sequences of SEQ ID NO:1-6. More specifically, administration of the pharmaceutical composition according to the invention will induce tolerance of the specific autoaggressive T cells to the autoantigens HAG end HCLP. The induced tolerance thus will lead to a reduction of the local inflammatory response in the articular cartilage under attack.

Very suitable peptides to be used in a pharmaceutical composition according to the invention are the peptides having 13-55, preferably 13-35, more preferably 13-25, highly preferably 13-19 amino acid residues charactenzed in that said peptides comprise at least one of the amino acid sequences given in SEQ ID NO:1 and 2.

Specifically preferred in a pharmaceutical composition according to the invention are the peptides having 13-55, preferably 13-35, more preferably 13-25, highly preferably 13-19 amino acid residues charactenzed in that said peptides compnse at least one of the amino acid sequences given in SEQ ID NO:3-6.

Highly preferred in a pharmaceutical composition according to the invention are peptides having 13 amino acid residues characterized in that said peptides have the amino acid sequence given in SEQ ID NO: 1 and 2.

Most preferred in a pharmaceutical composition according to the invention are the peptides having 13 amino acid residues with amino acid sequence of SEQ ID NO: 3, 4, 5 or 6.

The peptides according to the invention have the advantage that they have a specific effect on the autoreactive T cells thus leaving the other components of the immune system intact as compared to the nonspecific suppressive effect of the immunosuppressive steroid drugs. Treatment with the peptides according to the invention will be safe and no toxic side effects will occur.

Tolerance can be attained by administering high or low doses of peptides according to the invention. The amount of peptide will depend on the route of administration, the time of administration, the age of the patient as well as general health conditions and diet.

In general, a dosage of 0.01 to 1000 µg of peptide per kg body weight, preferably 0.5 to 500 µg, more preferably 0.1 to 100 µg of peptide can be used.

Pharmaceutical acceptable carriers are well known to those skilled in the art and include, for example, stenle salin, lactose, sucrose, calcium phosphate, gelatin, dextrin, agar, pectin, peanut oil, olive oil, sesame oil and water. Other carriers may be, for example MHC class II molecules, if desired embedded in liposomes.

In addition the pharmaceutical composition according to the invention may compnse one or more adjuvants. Suitable adjuvants include, amongst others, aluminium hydroxide, aluminium phosphate, amphigen, tocophenols, monophosphenyl lipid A, muramyl dipeptide and saponins such as Quill A. The amount of adjuvant depends on the nature of the adjuvant itself.

Furthermore the pharmaceutical composition according to the invention may comprise one or more stabilizers such as, for example, carbohydrates including sorbitol, mannitol, starch, sucrosedextrin and glucose, proteins such as albumin or casein, and buffers like alkaline phosphates.

Suitable administration routes are intramuscular injections, subcutaneous injections, intravenous injections or intrapentoneal injections, oral administration and nasal sprays.

The peptides according to the invention are also very suitable for use in a diagnostic method to detect the presence of activated autoreactive T cells involved in the chronic inflammation and destruction of the articular cartilage.

The diagnostic method according to the invention comprises the following steps:
a) isolation of the peripheral blood mononuclear cells (PBMC) from a blood sample of an individual,
b) culture said PBMC under suitable conditions,
c) incubation of said PBMC culture in the presence of the autoantigen or one or more peptides derived thereof according to the invention, and
d) detection of a response of T cells, for example a proliferative response, indicating the presence of activated autoreactive T cells in the individual.

The detection of a proliferative response of T cells can be detected by, for example, the incorporation of ³H-thymidine.

Also within the scope of the invention are test kits which comprise one or more peptides according to the invention. These test kits are suitable for use in a diagnostic method according to the invention.

The following examples are illustrative for the invention and should in no way be interpreted as limiting the scope of the invention.

### EXAMPLES

### METHODS

### Patients

Peripheral blood mononuclear cells (PBMC) from patients who were diagnosed as suffering from RA according to the Amencan Rheumatism Association (ARA) criteria (Arnett et al., Arthritis Rheum. 31:315, 1988) were collected. The seventy of disease of RA patients ranged from stage I - IV as determined by Röntgenscore. During the course of studies patients were treated with indomethacine, methotrexate, glucocorticoids or non-steroidal anti-inflammatory drugs

### MHC typing

Patient PBMC chromosomal DNA extracts were analysed using the Dynal DR 'low resolution' SSP kit. DR4 subtyping was performed using the Dynal DRB1*04-SSP kit. Interpretation of MHC typing data was done in collaboration with the University Transfusion service, Radboud hospital, Nijmegen, The Netherlands.

### Peptides

Peptides according to the invention and a control peptide IHA(307-319)F, PKFVKQNTLKAT (SEQ ID NO:7), were synthesized by solid-phase peptide synthesis. In brief, peptides with free amino- and carboxy termini were synthesized on a fully automated Milligen 9050 synthesizer, using Fmoc/tBu protected activated esters on PEG-PS resins. The peptides were cleaved off the resin and deprotected using TFA/Thioanisole/Ethanedithiol/Anisole 90/5/3/2 or TFA/H₂O 95/5 by volume. The peptides were purified by preparative HPLC, converted into acetate salts with Dowex Ac-resin or into chloride salts and lyophilized. Purity and identity of the peptides were asssessed by reverse phase HPLC and FAB-MS, respectively. The peptides used in this study are listed in Table 1. An N-terminal biotinylated Influenza Haemagglutinine denved peptide IHA(307-319)F, in which the third residue (Y) was replaced by F, (biotin-NH-(CH₂)₅-CO-PKFVKQNTLKLAT, SEQ ID No. 7), was used as marker peptide in the binding studies with DR4Dw4 (DRB1*0401). In order to prevent interference of the biotin group with peptide binding, biotin was attached via a spacer to the amino-terminus of the marker peptide.

**Table 1:**

| the peptides synthesized | | | |
|---|---|---|---|
| **Peptide** | **Sequence** | **HPLC punty** | **SEQ ID NO** |
| HAG1 | AGWLADQTVRYPI | > 90% | 3 |
| HAG2 | AGWLADRSVRYPI | > 90% | 4 |
| HAG3 | AGWLADGSLRYPI | > 90% | 5 |
| HCLP1 | AGWLSDGSVQYPI | > 90% | 6 |
| IHA(307-319)F | PKFVKQNTLKLAT | > 90% | 7 |

### Affinity purification of HLA-DR molecules

Two EBV-transformed B-cell lines, BSM (typed as DR4 [DRB1*0401] and BM92 (typed as DR4 [DRB1*0404) were provided by Dr. M. Oudshoom from the Academic Hospital Leiden, the Netherlands. The cells were cultured in Dulbecco's modified eagles minimal essential medium DMEM/HAM's F12 (Gibco Laboratories, Grand Island, NY) supplemented with 10% FCS (Hyclone Laboratories), 1% non-essential amino acids (ICI), L-glutamine, 2-ME and antibiotics. Cells were routinely passaged every 2-3 days in a 1:2 ratio. Cells were harvested and thereafter washed three times in phosphate buffer saline (PBS) (4°C) containing 1 mM PMSF. Cell pellets were stored at -70°C until use. HLA-DR expressing cells were thawed and lysed on ice for 30 minutes in PBS, 1% NP-40, 1 mM AEBSF (Calbiochem). The lysate was cleared of nuclei and debris by centrifugation at 15.000 rpm (Sorvall, SS34 rotor) for 30 minutes.

HLA-DR molecules were affinity purified from cell lysates using monoclonal antibody L243 (ATCC), directed against a nonpolymorphic determinant on the DR-complex (Lampson et al., J. Immunol. 125:293, 1980). Protein G sepharose purified L243 was coupled to NHS-Sepharose 4 FF (Pharmacia) according to the manufactures instructions. The cleared cell-lysate was passed through a 0.45 µm filter and added to L243-NHS-sepharose beads. After overnight incubation, the beads were transferred to a column and washed with five volumes PBS, 1% NP-40; 5 volumes PBS, 0.5% NP-40; 15 volumes PBS, 0.5% NP-40, 0.1% SDS; 5 volumes PBS, 0.05% NP-40; 5 volumes PBS, 1% n-octyl-glucoside (Sigma, St. Louis, USA) and 5 volumes 50 mM diethylamine (Fluka), 150 mM NaCI, 1% n-octyl-glucoside pH=8.0. HLA-DR molecules were eluted with 50 mM diethylamine, 150 mM NaCl, 1% n-octyl-glucoside pH=11. Immediately after collection, the fractions were neutralised with 2M glycine pH=4.8. Collected fractions were analysed on SDS-PAGE under non-reducing conditions followed by silver staining. Fractions containing purified HLA-DR were pooled and subsequently concentrated by ultrafiltration over a 30 kD cut-off membrane.

### Peptide HLA-DR binding assay

The peptide binding studies were performed using a semi-quantitative binding assay as described previously (Joosten et al., Int. Immunol. 6:751, 1994). Purified HLA-DR molecules (0.05-5 µM) were incubated at pH=5.0 with 50 nM biotinylated marker peptide (IHA(307-319)F) and a concentration range of competitor peptide (peptides HAG1, HAG2, HAG3 and HCLP1) in a final volume of 25 µl binding buffer (PBS, 1 mM AEBSF, 1 mM N-ethyl maleimide, 8 mM EDTA, 10 µM pepstatin A, 0.01% NaN₃, 0.05% NP-40 and 5% DMSO).

After approximately 48 hours incubation at room temperature, bound and unbound marker peptides were separated by SDS-PAGE under non-reducing conditions. Proteins were blotted onto a nitro-cellulose membrane (Hybond ECL, Amersham, U.K.) using a semi-dry blotting system (Pharmacia). The nitrocellulose filters were blocked with 0.5% DNA blocking reagents (Boehringer Mannheim, Germany) in 0.1 M maleic acid pH=7.5, 150 mM NaCI. After 1 hour, the filters were washed in PBS, 0.05% Tween 20 (Sigma, St. Louis, USA) and incubated with Streptavidin-HRP (Southem Biotechnology) in a 1:40.000 dilution. Biotinylated peptides were detected by enhanced chemoluminescence using a Westem Blot ECL kit (Amersham, U.K.) according to the manufactures instructions. Preflashed films (hyperfilm-ECL, Amersham, U.K.) were exposed for 30 minutes.

The affinity of a the peptides according to the invention for binding DAB1*0401 or DRB1*0404-encoded molecules was related to competition with the marker peptide. This relative binding affinity IC₅₀ (^{e}IC₅₀) was defined as the peptide concentration at which the signal of the biotinylated marker peptide was reduced to 50% by visual inspection of the signal in absence of the competitor peptide.

### Proliferative responses of blood mononuclear cells

The peptides HAG1, HAG2, HAG3 and HCLP1 were tested for their capacity to induce proliferative responses in PBMC. Proliferative assays involving PBMC have been used previously to measure activation of antigen-specific, class II restricted T-cell responses (Good et al, Proc. Natl. Acad.Sci USA 85: 1199, 1988)

PBMC obtained from heparinized venous peripheral blood were isolated by standard centrifugation on a Ficoll-Paque gradient. Cells were cultured in three- or fourfold at a concentration of 1,5 x 10⁵ cells/well in DMEM/Ham's F12 medium supplemented with 10% heat-inactivated pooled human serum, L-glutamine, 2-ME and antibiotics in flatbottomed microtiter plates. Cells were incubated in medium alone or in the presence of PHA (2.5 µg/ml) or in the presence of antigens, including the chicken proteoglycan fraction, the chicken collagen fraction, sonicated *Mycobacterium tuberculosis* or the peptides HAG1, HAG2, HAG3 and HCLP1 in concentrations of 50 µg/ml, 5µg/ml or 0.5 µg/ml. Cultures were incubated in a total volume of 210 µl for 4, 5, 6 or 7 days at 37 °C in a humidified atmosphere of 5% CO₂. Cultures were pulsed with 0.5µCi (1.85 x 10⁴ Bq) [³H]Thymidine ([³H]TdR) for the last 18 hours of cell culture. Cells were harvested on glassfibre filters and [3H]TdR incorporatiopn was measured by gasscintilation Note that counting by gasscintilation is fivefold less efficient compared to liquid scintilation. Therefor, filters were measured for 5 min (Packard Matrix 96 β-counter; Meriden CT).

### RESULTS

### Peptide HLA-DR binding assay

Binding of the peptides HAG1, HAG2, HAG3 and HCLP1 to DR4 (DRB1*0401) and DR4(DRB1*0404) was performed in a direct semi-quantitative competition binding assay using affinity purified HLA-DR molecules The reslts are shown in Table 2.. As can be seen from Table 2, all peptides bind to DR4(DAB1*0401) and are able to compete with the marker peptide.

**Table 2:**

| Peptide binding to HLA-DAB1*0401 encoded molecules | | |
|---|---|---|
| **Peptide** | **Sequence** | **DRB1*0401** ^{**e**}**IC**_{**50**} |
| HAG1 | AGWLADQTVRYPI | ++ |
| HAG2 | AGWLADRSVRYPI | ++ |
| HAG3 | AGWLADGSLRYPI | ++ |
| HCLP1 | AGWLSDGSVQYPI | + |
| ++ = good binder, ^{e}IC₅₀ between 1-10 µm; + = intermediate binder, ^{e}IC₅₀ between 10-100 µM; +/- = poor binder, ^{e}IC₅₀ between 100-1000 µM; - = non-binder, ^{e}IC₅₀ exceeds 1000 µM | | |

### PBMC proliferative assay

In order to determine T-cell reactivity to the peptides HAG1, HAG2, HAG3 and HCLP1, the PBMC proliferative response in RA patients was analyzed. Table 3 only represents the values obtained at 50 µg/ml. Results are expressed as the mean of three or four measurements (count per 5 min). Standard error of the mean did not exceed 30%. Underlined values are regarded positive (counts per 5 min > 1000 and stimulation index SI > 2)). Most patients responded to at least one of the peptides according to the invention, indicating that the peptides according to the invention are recognized by autoaggressive T-cells associated with autoimmune disease.

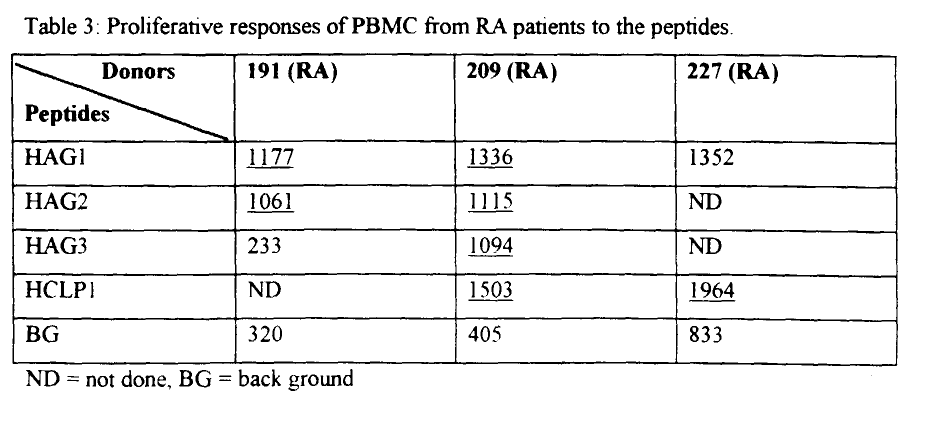

## Claims

1. Peptide having 13-55 amino acid residues characterized in that said peptide comprises the amino acid sequence
A G W L R₁ D R₂ R₃ R₄ R₅ Y P I
in which
R₁ = A, S
R₂ = Q, R, G
R₃ = T, S
R₄ = V, L
R₅ =R, Q.
with the provision that the peptide is not SSAGWLADRSVRYPISKARPNXGG, NAGWLSDGSVQYPITKPREP, DAGWLADGSVRYPISRPRKR, GGLDWCNAGWLSDGSVQYPITKPR or EQLFAAYEDGPEQCDAGWLADQTVRYPIRAPRVGCY.

2. Peptide having 13-55 amino acid residues characterized in that said peptide comprises at least the amino acid sequence
A G W L R₁ D R₂ R₃ L R₅ Y P I
in which
R₁ = A, S
R₂ = Q, R, G
R₃ = T, S
R₅ =R, Q.

3. Peptide having 13-55 amino acid residues characterized in that said peptide comprises at least one of the amino acid sequences AGWLADQTVRYPI, AGWLADRSVRYPI, AGWLSDGSVQYPI and AGWLADGSLRYPI, with the provision that the peptide is not SSAGWLADRSVRYPISKARPNXGG, NAGWLSDGSVQYPITKPREP, GGLDWCNAGWLSDGSVQYPITKPR or EQLFAAYEDGFEQCDAGWLADQTVRYPIRAPRVGCY.

4. Peptide consisting of one of the amino acid sequences AGWLADQTVRYPI, AGWLADRSVRYPI, AGWLSDGSVQYPI and AGWLADGSLRYPI.

5. Peptide having 13-55 amino acid residues and comprising the amino acid sequence A G W L R₁ D R₂ R₃ R₄ R₅ Y P I in which R₁ = A, S, R₂ = Q,R,G,R₃ = T, S, R₄ = V, L, and R₅ = R, Q, for use as a medicament.

6. Pharmaceutical preparation comprising a peptide having 13-55 amino acid residues, said peptide comprising the amino acid sequence A G W L R₁ D R₂ R₃ R₄ R₅ Y P I in which R₁= A, S, R₂ = Q, R G, R₃ = T, S, R₄ = V, L, and R₅ = R, Q, and a pharmaceutical acceptable carrier.

7. Pharmaceutical preparation comprising a peptide having 13-55 amino acid residues, said peptide comprising the amino acid sequence A G W L R₁ D R₂ R₃ L R₅ Y PI in in which R₁= A, S, R₂ = Q, R, G, R₃ = T, S and R₅ = R, Q, and a pharmaceutical acceptable carrier.

8. Pharmaceutical preparation comprising a peptide having 13-55 amino acid residues, said peptide comprising at least one of the amino acid sequences AGWLADQTVRYPI, AGWLADRSVRYPI, AGWLSDGSVQYPI and AGWLADGSLRYPI.

9. Pharmaceutical preparation comprising at least one of the peptides AGWLADQTVRYPI, AGWLADRSVRYPI AGWLADGSLRYPI and AGWLSDGSVQYPI and a pharmaceutical acceptable carrier.

10. Use of a peptide having 13-55 amino acid residues, said peptide comprising the amino acid sequence A G W L R₁ D R₂ R₃ R₄ R₅ Y PI in which R₁ = A, S, R₂ = Q, R, G, R₃ = T, S, R₄ = V, L, and R₅ = R, Q, for the manufacture of a pharmaceutical preparation for use in a peptide-induced tolerance therapy for the induction of tolarance to autoaggressive T cells associated with T-cell mediated articular cartilage destruction in autoimmune diseases.

## Patentansprüche

1. Peptid mit 13 bis 55 Aminosäureresten, dadurch gekennzeichnet, dass das genannte Peptid die Aminosäuresequenz
A G W L R₁ D R₂ R₃ R₄ R₅ Y P I
umfasst,
worin
R₁ = A, S
R₂ = Q, R, G
R₃ = T, S
R4 = V, L
R₅ = R, Q
ist,
unter der Bedingung, dass das Peptid nicht SSAGWLADRSVRYPISKARPMXGG, NAGWLSDGSVQYPITKPREP, DAGWLADGSVRYPISRPRKR, GGLDWCNAGWLSDGSVQYPITKPR oder EQLFAAYEDGFEQCDAGWLADQTVRYPIRAPRVGCY ist.

2. Peptid mit 13 bis 55 Aminosäureresten, dadurch gekennzeichnet, dass das genannte Peptid mindestens die Aminosäuresequenz
A G W L R₁ D R₂ R₃ L R₅ Y P I
umfasst,
worin
R₁ = A, S
R₂ = Q, R, G
R₃ = T, S
R₅ = R, Q
ist.

3. Peptid mit 13 bis 55 Aminosäureresten, dadurch gekennzeichnet, dass das genannte Peptid mindestens eine der Aminosäuresequenzen AGWLADQTVRYPI, AGWLADRSVRYPI, AGWLSDGSVQYPI und AGWLADGSLRYPI umfasst, unter der Bedingung, dass das Peptid nicht
SSAGWLADRSVRYPISKARPMXGG, NAGWLSDGSVQYPITKPREP, GGLDWCNAGWLSDGSVQYPITKPR oder EQLFAAYEDGFEQCDAGWLADQTVRYPIRAPRVGCY ist.

4. Peptid bestehend aus einer der Aminosäuresequenzen AGWLADQTVRYPI, AGWLADRSVRYPI, AGWLSDGSVQYPI und AGWLADGSLRYPI.

5. Peptid mit 13 bis 55 Aminosäureresten und umfassend die Aminosäuresequenz
A G W L R₁ D R₂ R₃ R₄ R₅ Y P I,
worin
R₁ = A, S
R₂ = Q, R, G
R₃ = T, S
R₄ = V, L und
R₅ = R, Q ist, zur Verwendung in einem Arzneimittel.

6. Pharmazeutisches Präparat, welches ein Peptid mit 13 bis 55 Aminosäureresten umfasst, wobei das genannte Peptid die Aminosäuresequenz A G W L R₁ D R₂ R₃ R₄ R₅ Y P I umfasst, worin
R₁ = A, S
R₂ = Q, R, G
R₃ = T, S
R₄ = V, L und
R₅ = R, Q
ist und einen pharmazeutisch annehmbaren Träger.

7. Pharmazeutisches Präparat, welches ein Peptid mit 13 bis 55 Aminosäureresten umfasst, wobei das genannte Peptid die Aminosäuresequenz A G W L R₁ D R₂ R₃ L R₅ Y P I umfasst,
worin
R₁ = A, S
R₂ = Q, R, G
R₃ = T, S
R₅ = R, Q
ist und einen pharmazeutisch annehmbaren Träger.

8. Pharmazeutisches Präparat, welches ein Peptid mit 13 bis 55 Aminosäureresten umfasst, wobei das genannte Peptid mindestens eine der Aminosäuresequenzen AGWLADQTVRYPI, AGWLADRSVRYPI, AGWLSDGSVQYPI und AGWLADGSLRYPI umfasst.

9. Pharmazeutisches Präparat, welches ein Peptid mit 13 bis 55 Aminosäureresten umfasst, wobei das genannte Peptid mindestens eine der Aminosäuresequenzen AGWLADQTVRYPI, AGWLADRSVRYPI, AGWLADGSLRYPI und AGWLSDGSVQYPI und einen pharmazeutisch anehmbaren Träger umfasst.

10. Verwendung eines Peptids mit 13 bis 55 Aminosäureresten, worin das genannte Peptid die Aminosäuresequenz A G W L R₁ D R₂ R₃ R₄ R₅ Y P I umfasst,
worin
R₁ = A, S
R₂ = Q, R, G
R₃ = T, S
R₄ = V, L
R₅ = R, Q ist, zur Herstellung eines pharmazeutischen Präparats zur Verwendung in einer Peptid-induzierten Toleranztherapie zur Induktion von Toleranz gegenüber auto-agressiven T-Zellen, die mit T-Zell-vermittelter Gelenkknorpelzerstörung bei Autoimmunkrankheiten in Zusammenhang stehen.

## Revendications

1. Peptide possédant de 13 à 55 résidus d'acides aminés, caractérisé en ce que ledit peptide comprend la séquence d'acides aminés
A GW L R₁ D R₂ R₃ R₄ R₅ Y PI
dans laquelle
R₁ = A, S
R₂ = Q, R, G
R₃ = T, S
R₄ = V, L
R₅ = R, Q
à condition que le peptide ne soit pas SSAGWLADRSVRYPISKARPNXGG, NAGWLSDGSVQYPITKPREP, DAGWLADGSVRYPISRPRKR, GGLDWCNAGWLSDGSVQYPITKPR ni EQLFAAYEDGFEQCDAGWLADQTVRYPIRAPRVGCY.

2. Peptide possédant de 13 à 55 résidus d'acides aminés, caractérisé en ce que ledit peptide comprend la séquence d'acides aminés
A G W L R₁ D R₂ R₃ L R₅ Y P I
dans laquelle
R₁ = A, S
R₂ = Q, R, G
R₃ = T, S
R₅ - R, Q

3. Peptide possédant de 13 à 55 résidus d'acides aminés, caractérisé en ce que ledit peptide comprend au moins l'une des séquences d'acides aminés AGWLADQTVRYTI, AGWLADRSVRYPI, AGWLSDGSVQYPI et AGWLADGSLRYPI,
à condition que le peptide ne soit pas SSAGWLADRSVRYPISKARPNXGC, NAGWLSDGSVQYPITKPREP, GGLDWCNAGWLSDGSVQYPITKPR ni FQLFAAYEDGFEQCDAGWLADQTVRYPIRAPRVGCY.

4. Peptide consistant en l'une des séquences d'acides aminés AGWLADQTVRYPI, AGWLADRSVRYPI, AGWLSDGSVQYPI et AGWLADGSLRYPI.

5. Peptide possédant de 13 à 55 résidus d'acides aminés et comprenant la séquence d'acides aminés A G W L R₁ D R₂ R₃ R₄ R₅ Y P I, dans laquelle R₁ = A, S ; R₂ = Q, R, G R₃ = T, S, R₄ = V, L; et R₅ = R, Q; destiné à être utilisé comme médicament.

6. Préparation pharmaceutique comprenant un peptide possédant de 13 à 55 résidus d'acides aminés, ledit peptide comprenant la séquence d'acides aminés A G W L, R₁ D R₂ R₃ R₄ R₅ Y P I, dans laquelle R₁ = A, S ; R₂ = Q, R, G; R₃ = T, S ; R₄ = V, L ; et R₅ = R, Q ; et un véhicule pharmaceutique acceptable.

7. Préparation pharmaceutique comprenant un peptide possédant de 13 à 55 résidus d'acides aminés, ledit peptide comprenant la séquence d'acides aminés A G W L R₁ D R₂ R₃ L R₅ Y P I, dans laquelle R₁ = A, S , R₂ = Q, R, G; R₃ = T, S ; et R₅ = R, Q ; et un véhicule pharmaceutique acceptable.

8. Préparation pharmaceutique comprenant un peptide possédant de 13 à 55 résidus d'acides aminés, ledit peptide comprenant au moins l'une des séquences d'acides aminés AGWLADQTVRYPI, AGWLADRSVRYPI, AGWLSDGSVQYPI et AGWLADGSLRYPI.

9. Préparation pharmaceutique comprenant au moins l'un des peptides AGWLADQTVRYPI, AGWLADRSVRYPI, AGWLADGSLRYPI et AGWLSDGSVQYPI et un véhicule pharmaceutique acceptable.

10. Utilisation d'un peptide possédant de 13 à 55 résidus d'acides aminés, ledit peptide comprenant la séquence d'acides aminés A G W L, R₁ D R₂ R₃ R₄ R₃ Y P I. dans laquelle R₁ = A, S ; R₂ = Q, R, G; R₃ = T, S , R₄ = V, L; et R₅ = R, Q, pour la fabrication d'une préparation pharmaceutique destinée à être utilisée dans une thérapie de tolérance induite par des peptides destinée à induire une tolérance aux lymphocytes T auto-agressifs associés à la destruction du cartilage dont la médiation est assurée par les lymphocytes T dans les maladies auto-immunes.


### SEQUENCE LISTING

### (1) GENERAL INFORMATION:

(i) APPLICANT:
   (A) NAME: AKZO NOBEL N.V.
   (B) STREET: Velperweg 76
   (C) CITY: Arnhem
   (E) COUNTRY: The netherlands
   (F) POSTAL CODE (ZIP): 6824 BM
   (G) TELEPHONE: 04120-66376
   (H) TELEFAX: 04120-50592
   (I) TELEX: 37503 akpha nl
(ii) TITLE OF INVENTION: Novel Peptides For Use In Treatment Of T-Cell Mediated Cartilage Destruction In Autoimmune Diseases
(iii) NUMBER OF SEQUENCES: 7
(iv) COMPUTER READABLE FORM:
   (A) MEDIUM TYPE: Floppy disk
   (B) COMPUTER: IBM PC compatible
   (C) OPERATING SYSTEM: PC-DOS/MS-DOS
   (D) SOFTWARE: PatentIn Release #1.0, Version #1.25 (EPO)

### (2) INFORMATION FOR SEQ ID NO: 1:

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(ix) FEATURE:
   (A) NAME/KEY: Peptide
   (B) LOCATION: 1..13
   (D) OTHER INFORMATION: /label= Xaa /note= "Xaa on pos 5 = A or S; Xaa on pos 7 = Q, R or G; Xaa on pos 8 = T or S; Xaa on pos 9 = V or L; Xaa on pos 10 = R or Q"
(xi) SEQUENCE DESCRIPTION: SEQ ID NO: 1:
   Ala Gly Trp Leu Xaa Asp Xaa Xaa Xaa Xaa Tyr Pro Ile
   1               5                   10

### (2) INFORMATION FOR SEQ ID NO: 2:

(1) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(ix) FEATURE:
   (A) NAME/KEY: Peptide
   (B) LOCATION: 1..13
   (D) OTHER INFORMATION: /label= PEPTIDE /note= "Xaa on pos 5 = A or S; Xaa on pos 7 = Q, R org; Xaa on pos 8 = T or s; Xaa on pos 10 = R or Q"
(xi) SEQUENCE DESCRIPTION: SEQ ID NO: 2:
   Ala Gly Trp Leu Xaa Asp Xaa Xaa Leu Xaa Tyr Pro Ile
   1               5                   10

### (2) INFORMATION FOR SEQ ID NO: 3:

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(xi) SEQUENCE DESCRIPTION: SEQ ID NO: 3:
   Ala Gly Trp Leu Ala Asp Gln Thr Val Arg Tyr Pro Ile
   1               5                   10

### (2) INFORMATION FOR SEQ ID NO: 4:

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(xi) SEQUENCE DESCRIPTION: SEQ ID NO: 4:
   Ala Gly Trp Leu Ala Asp Arg Ser Val Arg Tyr Pro Ile
   1               5                   10

### (2) INFORMATION FOR SEQ ID NO: 5:

(i) SEQUENCE CHARACTERISTICS:
   (A) LENGTH: 13 amino acids
   (B) TYPE: amino acid
   (C) STRANDEDNESS: single
   (D) TOPOLOGY: linear
(11) MOLECULE TYPE: peptide
(iii) HYPOTHETICAL: NO
(xi) SEQUENCE DESCRIPTION: SEQ ID NO: 5:
   Ala Gly Trp Leu Ala Asp Gly Ser Leu Arg Tyr Pro Ile
   1               5                   10

### (2) INFORMATION FOR SEQ ID NO: 6:

(1) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(xi) SEQUENCE DESCRIPTION: SEQ ID NO: 6:
   Ala Gly Trp Leu Ser Asp Gly Ser Val Gln Tyr Pro Ile
   1               5                   10

### (2) INFORMATION FOR SEQ ID NO: 7:

(i) SEQUENCE CHARACTERISTICS:
   (A) LENGTH: 13 amino acids
   (B) TYPE: amino acid
   (C) STRANDEDNESS: single
   (D) TOPOLOGY: linear
(ii) MOLECULE TYPE: peptide
(iii) HYPOTHETICAL: NO
(xi) SEQUENCE DESCRIPTION: SEQ ID NO: 7:
   Pro Lys Phe Val Lys Gln Asn Thr Leu Lys Leu Ala Thr
   1               5                   10